# EUROPEAN PATENT SPECIFICATION

(11) **EP 1 237 603 B1**
(45) Date of publication and mention of the grant of the patent: **01.09.2004**
(21) Application number: 00978188.1
(22) Date of filing: 24.11.2000
(51) Int. Cl.: A61M 11/00, A61M 15/00, G06M 1/00

(54) **COUNTING MECHANISM, AND SPRAY DOSE INHALER WITH SAID COUNTING MECHANISM**
ZÄHLMECHANISMUS SOWIE DOSIER INHALATOR MIT DESSEM MECHANISMUS
MECANISME DE COMPTAGE ET INHALATEUR POUR PULVERISATION DE DOSES EQUIPE DE CE MECANISME

(30) Priority: 26.11.1999 SE 9904278
(43) Date of publication of application: 11.09.2002
(73) Proprietor: Ernst Hörlins Ingenjörsbyra AB, 436 39 Askim (SE)
(72) Inventor: HÖRLIN, Ernst, S-436 39 Askim (SE)
(74) Representative: Romare, Laila Anette
(86) International application number: PCT/SE2000/002319
(87) International publication number: WO 2001/037909

(56) References cited:
- WO-A1-86/02275
- DE-C1- 19 549 033

## Description

The invention relates to a counting mechanism for counting the number of doses delivered from a container and comprising means for connection to the container, for which the closest prior art is WO-A-8602275. The invention also concerns a spray dose inhaler into which a spray container can be introduced and comprising a counting mechanism for counting the number of doses delivered from the spray container.

### BACKGROUND ART

It has become increasingly common for medicines to be administered by being sprayed into the mouth or nose. For this purpose, spray dose inhalers have been developed which deliver one dose of medication per spray. However, a considerable problem with the spray dose inhalers which have hitherto been made available is that they do not have means for indicating how many doses remain in the spray container. This means that many spray containers are discarded long before they are empty, and in many cases even when they are only half empty. There is therefore a great need for a counting mechanism in order to be able to monitor the spray consumption in a spray dose inhaler.

However, it has proven very difficult to obtain a reliable counting mechanism which does not adversely affect the function of the inhaler since spray administration is a method which is very sensitive to external disturbances. A counting mechanism for a spray dose inhaler must therefore have a structure which does not cause variations in the size of the delivered doses, which does not create spaces where the active substance can accumulate, and which does not in any other way interfere with the function of the inhaler.

No satisfactory solution to the above problems has as yet been put forward.

### DISCLOSURE OF THE INVENTION

The present invention nevertheless makes available a counting mechanism which is of the type discussed in the introduction and which largely avoids the problems mentioned above. An arrangement according to the invention is mainly characterized in that the counting mechanism comprises a cap, a counting wheel, and a control wheel, all of which have a substantially circular shape and are concentric in relation to each other, the cap having an outer surface and an inner surface, and a flange-like cap edge projecting from the periphery of the inner surface, and comprising a gear ring which is arranged inside the flange-like cap edge on the inner surface of the cap, an indicator window in the form of a through-opening in the cap, two concentrically arranged inner flanges which project centrally from the inner surface of the cap and of which a first inner flange is arranged radially inside a second inner flange and projects a distance beyond the second inner flange, and in that the first inner flange has a radially projecting locking tooth, and the counting wheel has an outer surface and an inner surface and is arranged directly next to the cap with the outer surface of the counting wheel directed towards the inner surface of the cap, the counting wheel comprising a centrally arranged through-hole with a shape and size corresponding to the shape and size of the second inner flange of the cap, at least one stop tongue which projects from the periphery of the counting wheel and is arranged to engage in and cooperate with the gear ring arranged on the inner surface of the cap, and a gear ring arranged on the inner surface of the counting wheel, near the periphery of the counting wheel, and a measurement scale arranged on the outer surface and placed so that it is visible through the indicator window, and in that the control wheel has an outer surface and an inner surface and is arranged directly next to the counting wheel, with the outer surface of the control wheel directed towards the inner surface of the counting wheel, the control wheel comprising a centrally arranged through-hole with a shape and size corresponding to the shape and size of the first inner flange of the cap and having a recess arranged on the periphery of the hole, which recess has a shape which substantially corresponds to the shape of the locking tooth arranged on the cap and with a size which slightly exceeds the size of the locking tooth, so that a predetermined clearance exists between the locking tooth and the edges of the recess, at least one stop tongue which projects from the periphery of the control wheel and which is arranged to engage in and cooperate with the gear ring arranged on the inner surface of the counting wheel, and a rod-shaped pin which is arranged at right angles to the inner surface of the control wheel and is connected to the control wheel via a spring member, and in that the stop tongue of the control wheel is oriented in a tangential direction which is opposite to the direction of the gear ring and stop tongue of the counting wheel, and in that the direction of the teeth in the gear ring of the cap coincides with the tangential direction of the stop tongue of the control wheel, and furthermore in that the number of stop tongues and the number of teeth are identical for the counting wheel and for the control wheel.

Although not essential to the invention, it may be expedient, for production reasons and assembly reasons, that the first and second inner flanges of the cap, and the centrally arranged through-holes in the counting wheel and control wheel, are substantially circular with a radius corresponding to the radius of the first and second inner flange, respectively, of the cap.

It is also advantageous for the stop tongues on the counting wheel and control wheel to be arranged to spring in the radial direction, as a result of which the stop tongues bear with a certain spring force against the teeth of the gear rings of the counting wheel and cap. Radial direction is understood as a radial direction in towards or out from a centre axis running through the counting mechanism.

The number of doses which the counting mechanism is able to count depends on the clearance between the locking tooth, arranged on the inside of the cap, and the corresponding recess in the control wheel, and also on the number of teeth in the gear rings of the counting wheel and cap. For counting mechanisms intended to count up to 130 doses, the clearance should be about 3° and the number of teeth should be 46. For counting mechanisms intended to count 130 to 240 doses, the clearance should be 1.5° and the number of teeth should be 82. The clearance is calculated by dividing 360° by the number of doses which the counting mechanism is intended to be able to count, after which a whole 1° is added to the result. The number of teeth must be evenly divisible by the number of stop tongues, plus one extra tooth.

A suitable spring member for connecting the rod-shaped pin to the control wheel, and for transferring forces acting on the rod-shaped pin to the control wheel, is a flat spring. The spring member can be a separately manufactured component, but it is advantageously made integral with the rod-shaped pin and the control wheel, for example by injection-moulding of plastic.

The invention also concerns a complete spray dose inhaler of the type discussed in the introduction. A spray dose inhaler according to the invention is characterized mainly in that the counting mechanism is arranged substantially on the outside of the inhaler and comprises a cap, a counting wheel, and a control wheel, all of which have a substantially circular shape and are concentric in relation to each other, the cap having an outer surface and an inner surface, and a flange-like cap edge projecting from the periphery of the inner surface, and comprising a gear ring which is arranged inside the flange-like cap edge on the inner surface of the cap, an indicator window in the form of a through-opening in the cap, two concentrically arranged inner flanges which project centrally from the inner surface of the cap and of which a first inner flange is arranged radially inside a second inner flange and projects a distance beyond the second inner flange, and in that the first inner flange has a radially projecting locking tooth, and the counting wheel has an outer surface and an inner surface and is arranged directly next to the cap with the outer surface of the counting wheel directed towards the inner surface of the cap, the counting wheel comprising a centrally arranged through-hole with a shape and size corresponding to the shape and size of the second inner flange of the cap, at least one stop tongue which projects from the periphery of the counting wheel and is arranged to engage in and cooperate with the gear ring arranged on the inner surface of the cap, and a gear ring arranged on the inner surface of the counting wheel, near the periphery of the counting wheel, and a measurement scale arranged on the outer surface and placed so that it is visible through the indicator window, and in that the control wheel has an outer surface and an inner surface and is arranged directly next to the counting wheel, with the outer surface of the control wheel directed towards the inner surface of the counting wheel, the counting wheel comprising a centrally arranged through-hole with a shape and size corresponding to the shape and size of the second inner flange of the cap and having a recess arranged on the periphery of the hole, which recess has a shape which substantially corresponds to the shape of the locking tooth arranged on the cap and with a size which is slightly greater than the locking tooth, so that a predetermined clearance exists between the locking tooth and the edges of the recess, at least one stop tongue which projects from the periphery of the control wheel and which is arranged to engage in and cooperate with the gear ring arranged on the inner surface of the counting wheel, and a rod-shaped pin which is arranged at right angles to the inner surface of the control wheel and is connected to the control wheel via a spring member, and in that the stop tongue of the control wheel is oriented in a tangential direction which is opposite to the direction of the gear ring and stop tongue of the counting wheel, and in that the direction of the teeth in the gear ring of the cap coincides with the tangential direction of the stop tongue of the control wheel, and furthermore in that the number of stop tongues and the number of teeth are identical for the counting wheel and for the control wheel, and in that the outside of the spray dose inhaler is provided with coupling members for connecting the counting mechanism to the spray dose inhaler, the coupling members comprising a through-hole through which the rod-shaped pin of the counting mechanism can be introduced, the hole being positioned so that the rod-shaped pin is in contact with the spray container and is acted upon and deflected and/or changes position when the spray container moves in the first part of the spray dose inhaler.

Since the invention allows the counting mechanism to be arranged almost entirely on the outside of the inhaler, with only the very small rod-shaped pin situated inside the inhaler, the counting mechanism by and large does not in any way affect the flow through the inhaler. This means that the doses delivered retain a constant size, and that the risk of active substance accumulating in the inhaler as a result of the action of the counting mechanism is practically nonexistent.

The dose scale arranged on the counting wheel is placed in an annular configuration on the outside of the counting wheel and is either ascending or descending, depending on whether the counting mechanism is to indicate how many doses have been administered or how many doses remain. The dose scale is preferably a numerical scale, with the dose number being indicated in steps of 1, 2, 5, 10, 25 doses or in other dose steps if deemed appropriate. Alternatively, or in addition to this, it is however possible to imagine using a colour scale, for example ranging from green through yellow to red. For example, the last 10% of the doses can be indicated by red or another differing colour. As the counting wheel is turned round during use, new parts of the dose scale successively appear through the indicator window in the counting mechanism cap. The indicator window is preferably provided with an arrow which points to the dose number in each position.

The counting mechanism can be provided with a built-in stop position. Such a counting mechanism is discarded when it has counted the predetermined number of doses for which it has been designed. It is also possible to produce re-usable counting mechanisms without built-in stop devices. Such a stop device can be provided, for example, by arranging a pin on the inside of the cap, which pin runs in a groove in the counting wheel.

### BRIEF DESCRIPTION OF THE FIGURES

The invention will be described in greater detail below with reference to the illustrative embodiments shown in the attached figures, where:
Figure 1 shows a spray dose inhaler with a counting mechanism according to the invention;
Figures 2a and 2b show an outer casing for a counting mechanism according to the invention;
Figures 3a and 3b show a counting wheel for a counting mechanism according to the invention;
Figures 4a and 4b show a control wheel for a counting mechanism according to the invention;
Figure 5 shows an exploded view of a spray dose inhaler with associated components;
Figure 6 shows a spray dose inhaler with an attachment arrangement for a counting mechanism according to the invention;
Figure 7 shows a cross section through a spray dose inhaler and a counting mechanism according to the invention; and
Figure 8 shows an exploded view of a counting mechanism according to the invention.

### DETAILED DESCRIPTION OF EMBODIMENTS

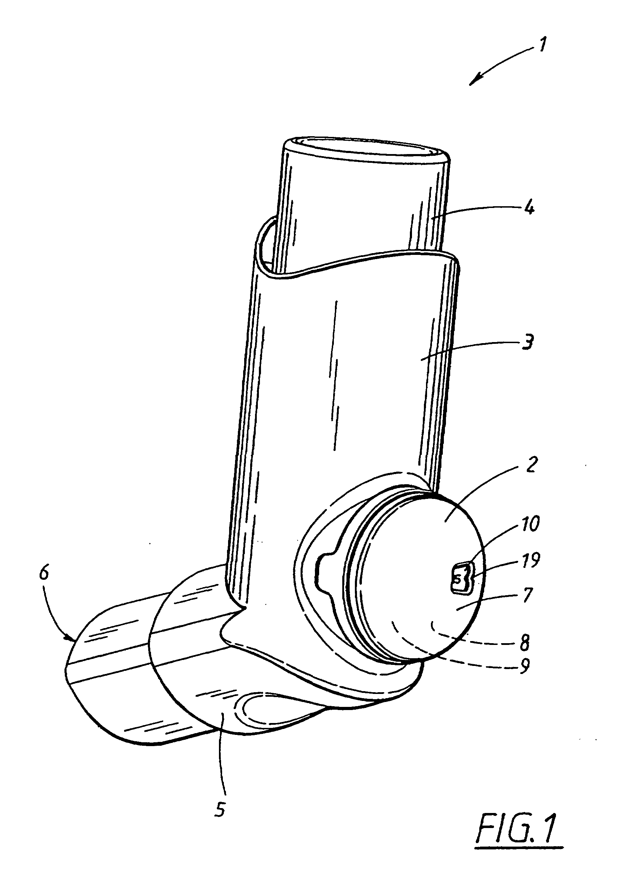

Figure 1 shows a spray dose inhaler 1 which is provided with a counting mechanism 2 arranged substantially on the outside of the inhaler. The spray dose inhaler 1 has an upper tubular part 3 into which a spray container 4 is introduced. The spray dose inhaler 1 also has a lower tubular mouthpiece part 5 which is angled in relation to the upper part 3 and which emerges via an opening 6 from which the contents of the spray container 4 are delivered. When using the spray dose inhaler 1, the mouthpiece part 5 is placed in the user's mouth and the spray container 4 is pressed further down in the upper part 3 of the inhaler 1. A dose is thereby released and is sprayed into the user's oral cavity.

The counting mechanism 2 consists of three main components, namely a cap 7, a counting wheel 8, and a control wheel 9. Arranged on the outside is the substantially circular cap 7, the latter thus serving as an outer casing for the counting mechanism 2. The cap 7 is provided with an indicator window 10, and on the inside of the cap 7 there is a gear ring 11 in which the number of teeth 12 is adapted to the number of doses in the spray container.

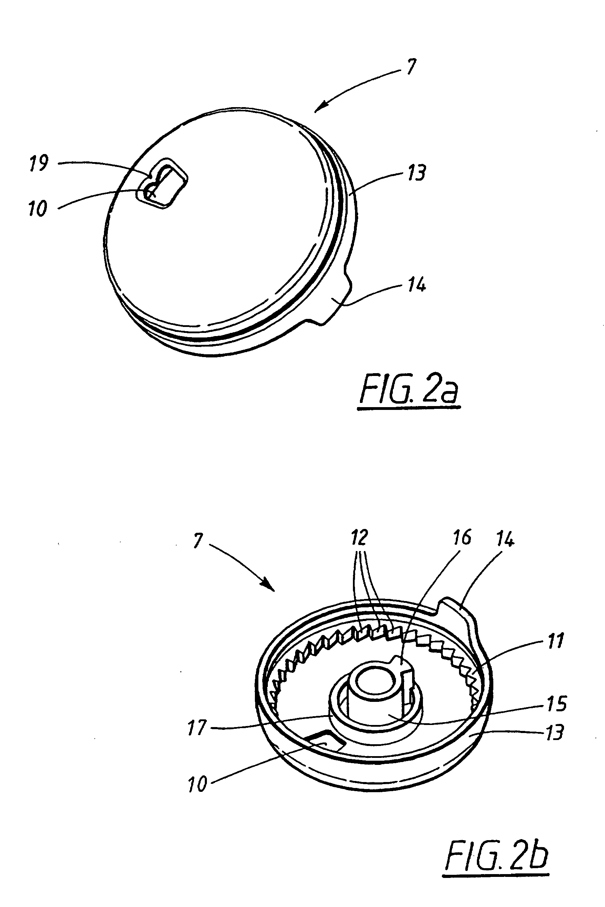

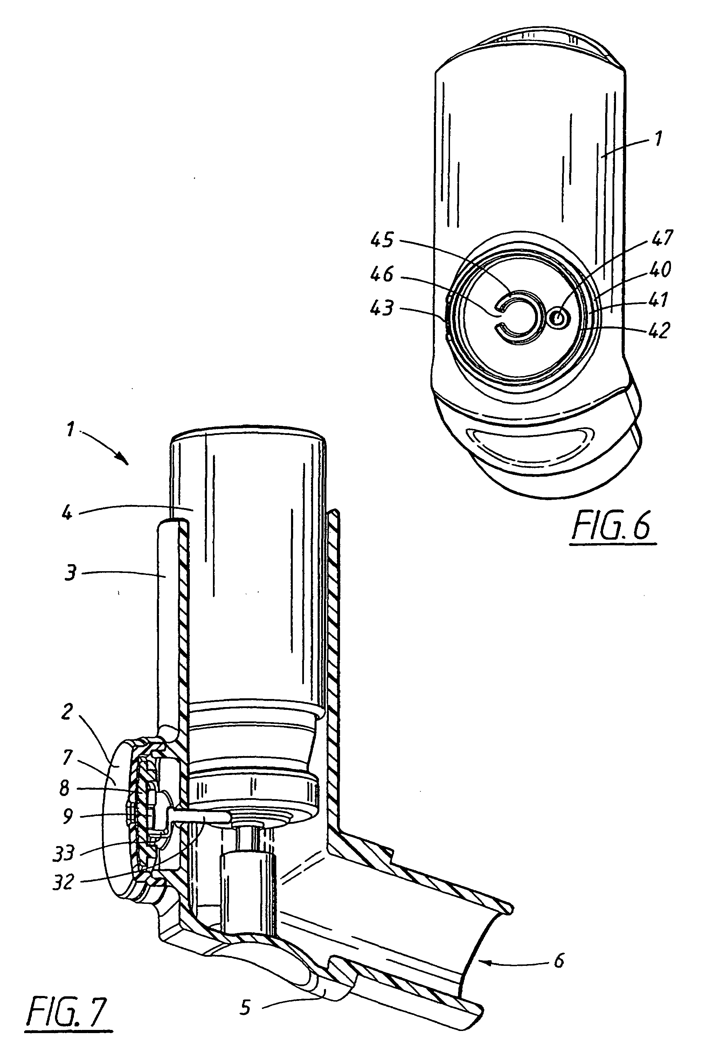

Such a cap is shown in Figures 2a and 2b. Figure 2a shows the outside of the cap 7, i.e. the side directed away from the inhaler when the counting mechanism is fitted on an inhaler, and Figure 2b shows the inside of the cap, i.e. the side directed towards the inhaler when the counting mechanism 2 is fitted. The teeth 12 are inclined and slope in a first tangential direction. In Figure 2a the teeth 12 are shown with an inclination anti-clockwise in the figure. The cap 7 is additionally provided with a coupling arrangement comprising several parts and intended to cooperate with a corresponding coupling arrangement on the inhaler 1. The coupling arrangement on the inhaler 1 is shown in detail in Figure 6 and will be described in detail in the description of Figure 6. The coupling arrangement on the cap 7 comprises, on the one hand, a cap edge 13 projecting at right angles from the plane of the cap 7 and an almost rectangular pin 14 projecting from the cap edge 13, and, on the other hand, a centrally arranged cylindrical first inner flange 15. Moreover, the cylindrical inner flange 15 has a locking tooth 16 projecting radially from the outer surface of the inner flange 15.

Arranged concentrically to the outside of the cylindrical inner flange 15 there is an annular second inner flange 17 which has less height above the inner surface of the cap than does the cylindrical first inner flange 15, and which is intended to serve as coupling member between the cap 7 and the counting wheel 8. The cylindrical first inner flange 15 here fulfills the additional purpose of being a coupling member between the cap 7 and the control wheel 9.

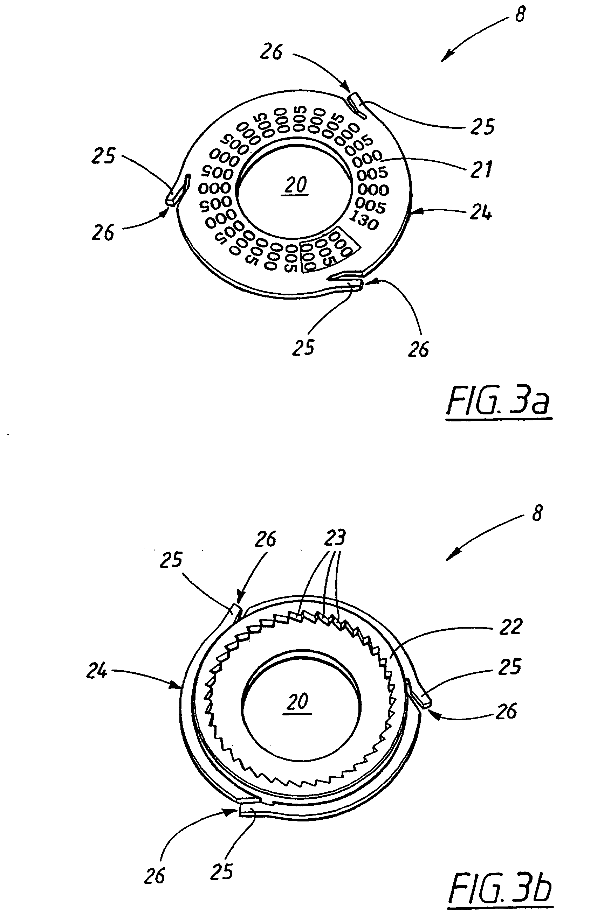

Arranged inside of the cap 7. or outer casing, there is a counting wheel 8 of the type shown in Figures 3a and 3b. The counting wheel 8 has a substantially circular shape, with a central and circular through-hole 20. In the same way as in Figures 2a and 2b, the counting wheel 8 in Figure 3a is shown from the outside, which is the side directed outwards and away from the inhaler 1, and Figure 3b shows the inside, which is the side of the counting wheel 8 directed inwards and towards the inhaler 1. As can be seen from Figure 3a, the outside of the counting wheel 8 is provided with a numerical scale 21 which is intended to continuously indicate the number of doses remaining in the spray container 4. In the illustrative embodiment shown, the doses are indicated in intervals of 5. In some cases it can of course be expedient to count the doses in another way, for example in individual doses or in intervals of two doses, ten doses, 25 doses, and so on, depending on how many doses in total are present in the spray container 4. The counting wheel 8 is either numbered in ascending sequence, so that the number of doses used is indicated, or in descending sequence, so that the number of doses remaining is indicated. The numerical scale 21 is visible through the indicator window 10 on the cap. As can be seen for example from Figure 2, the indicator window 10 can be provided with an indicator arrow 19 which points to the current number of doses. Moreover, the last doses, for example the last 10% of the doses, are marked in a different way, preferably with a different colour such as red.

As with the cap 7, a gear ring 22 is arranged on the inside of the counting wheel 8, about its periphery. The number of teeth 23 is adapted to the number of doses which are to be counted, and the teeth 23 are oriented in the opposite direction in relation to the teeth 12 on the cap 7, which means that the teeth 23 in Figure 3b slope clockwise. In addition, the counting wheel 8 is provided with three stop tongues 25 arranged symmetrically about the outer edge 24 of the counting wheel 8. The stop tongues 25 are designed in such a way that they have a certain spring capacity, upon radial loading, in towards or out from the centre of the counting wheel 8. As will be observed in Figures 3a and 3b, the stop tongues 25 have a free pin-shaped part which is directed along the periphery of the counting wheel 8 and which ends in a stop edge 26. In the example shown, the free parts of the stop tongues 25, as can be seen from Figure 3b, are directed clockwise about the periphery of the counting wheel 8. The stop tongues 25 thus have the same direction as the teeth 23 of the counting wheel 8.

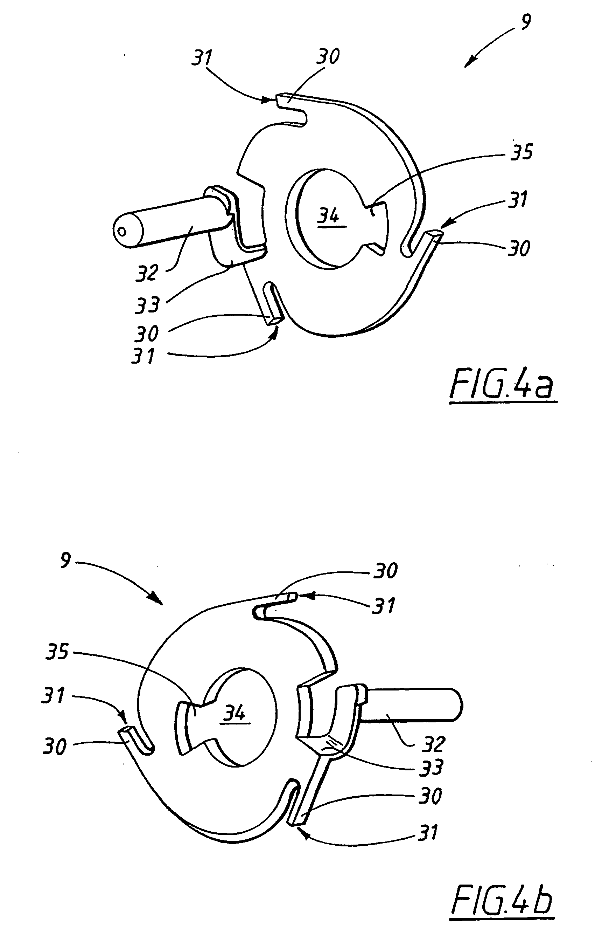

The third part of the counting mechanism 2 is the control wheel 9 shown in Figures 4a and 4b. The control wheel 9 is also substantially circular in shape and is provided with three resilient stop tongues 30 which are distributed symmetrically about the periphery of the wheel 9 and have a free part which springs radially and ends in a stop edge 31. However, the stop tongues 30 of the control wheel 9 are directed in the opposite direction in relation to the stop tongues 25 of the counting wheel 8. In addition, the control wheel 9 has a rod-shaped pin 32 which is arranged at right angles to the inside of the control wheel 9, which is the side of the control wheel 9 intended to be directed towards the inhaler 1. The pin 32 is connected to the control wheel 9 via a flat spring member 33. A circular hole 34 is arranged centrally through the control wheel 9 and has a recess 35 arranged on the periphery of the hole 34, which recess 35 has a shape corresponding substantially to the shape of the locking tooth 16 arranged on the cap 7. When the counting mechanism 2 is assembled, the locking tooth 16 is in fact intended to be introduced into the recess 35 of the control wheel 9, in the manner of a key in a lock. As has previously been mentioned, it is intended that there should be a certain clearance or play between the locking tooth 16 and the edges of the recess 35, which clearance has a predetermined size and contributes to defining the size of the counting steps which the counting mechanism 2 registers.

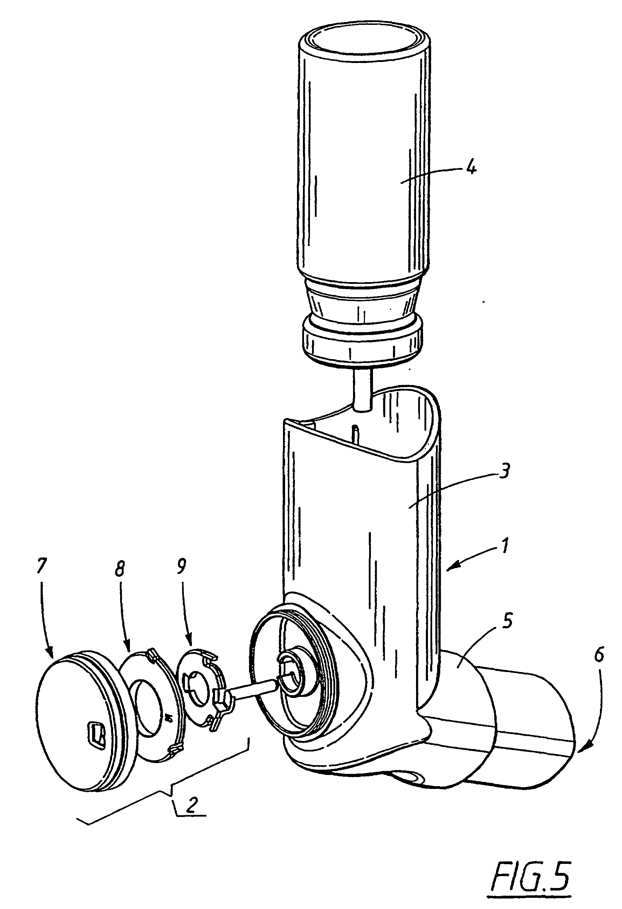

Figure 5 shows how the different parts are joined together to form a complete inhaler 1 with counting mechanism 2 and spray container 4. The spray container 4 is introduced into the upper tubular part 3 of the spray dose inhaler 1, and the counting mechanism 2 is fitted on the outside of the upper tubular part 3 of the spray dose inhaler 1, near the transition to the mouthpiece part 5.

Figure 6 shows in detail the appearance of the coupling arrangement provided on the spray dose inhaler 1. The coupling arrangement comprises a circular outer flange 40, which projects from the surface of the inhaler 1 and which has an outer ring 41 and also an inner ring 42 which projects farther than the outer ring 41 and has an external radius corresponding to the internal radius of the projecting cap edge 13 on the cap 7 of the counting mechanism 2. A recess 43 is also provided in the outer ring 41 for cooperation with the substantially rectangular pin 14 arranged on the cap edge 13. Thus, the counting mechanism 2 is fitted with the cap 7 tight over the outer ring 41 of the coupling arrangement of the spray dose inhaler 1, and with the pin 14 on the cap edge 13 inserted into the recess 43 on the outer ring 41 of the coupling arrangement.

The spray dose inhaler 1 also has a centrally arranged annular inner flange 45 whose internal diameter corresponds to the external diameter of the cylindrical first inner flange 15 on the counting mechanism cap 7. The annular inner flange 45 arranged on the spray dose inhaler 1 has a gap 46 through which the locking tooth 16 arranged on the cylindrical first inner flange 15 of the counting mechanism cap 7 can project.

The spray dose inhaler 1 finally has a through-hole 47 arranged between the inner flange 45 and outer flange 40 of the coupling arrangement. The through-hole 47 is designed such that the rod-shaped pin 32 arranged on the control wheel 9 can be introduced through the wall of the spray dose. inhaler 1 and into the inside of the spray dose inhaler. The hole has a diameter which is substantially the same as the diameter of the rod-shaped pin 32. Although the hole 47 and the rod-shaped pin in the illustrative embodiment shown have a circular cross section, it is possible, within the scope of the invention, to use holes/pins with edged or oval cross sections.

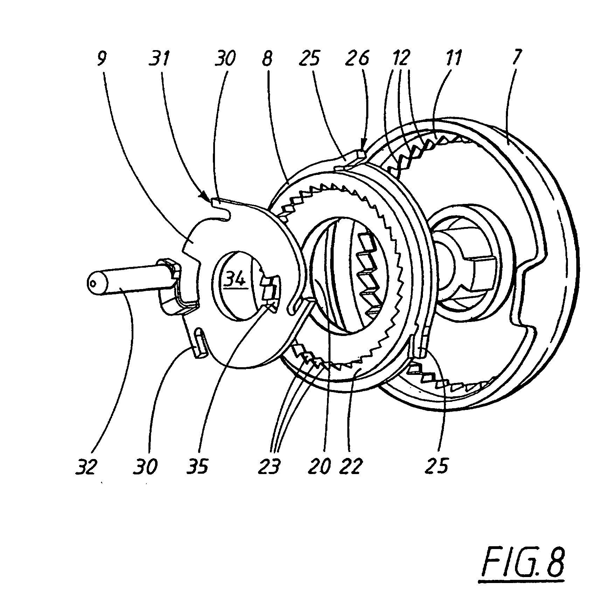

Figure 7 shows how the spray dose inhaler 1 looks when the counting mechanism 2 has been fitted and the spray container 4 has been introduced into the upper tubular part 3 of the inhaler 1. The rod-shaped pin 32 of the control wheel 9 is introduced through the hole 47 in the inhaler wall, and the hole 47 is positioned in such a way that the pin 32 is thereby in contact with the spray container 4 in such a way that, when the spray container 4 is pressed into the inhaler 1, the spray container 4 presses the pin 32 in its direction of movement. In so doing, that part of the pin 32 located inside the inhaler 1 is deflected and/or angled in the downward direction in Figure 7. This means that the flat spring 33 connecting the pin 32 to the control wheel 9 acts on the control wheel 9 in a direction opposite to the direction of movement of the pin 32 and spray container 4. The cooperation between the various parts of the counting mechanism 2 will be readily understood by referring to Figure 8, from which figure it clearly emerges how the directions of movement of the different gear rings 11, 22 and stop tongues 25, 30 relate to each other. When the rod-shaped pin 32 is pressed downwards in the inhaler (as is shown in Figure 7), the control wheel 9 is therefore rotated clockwise, viewed axially in the direction from within the inhaler 1. However, the rotation can only occur as far as is permitted by the clearance between the locking tooth 16 of the cap 7 and the corresponding recess 35 on the control wheel 9. By means of this rotation, one of the three stop tongues 30 arranged on the control wheel is additionally moved to a new position between two teeth 23 of the counting wheel 8. When the load exerted by the spray container 4 on the rod-shaped pin 32 ceases, the pin 32 returns to the substantially horizontal position shown in Figure 7 and the control wheel 9 springs back to its initial position, which corresponds to the control wheel 9 rotating anti-clockwise as far as is permitted by the clearance between the locking tooth 16 of the cap 7 and the corresponding recess 35 on the control wheel 9. During this movement, one of the stop tongues 30 of the control wheel 9 rests with its outer stop edge 31 against a tooth 23 of the counting wheel 8. In this way, the return movement is transmitted to the counting wheel 8 which thus rotates anti-clockwise and counts one step, which can be observed in the indicator window 10 of the cap 7. At the same time, one of the locking tongues 25 on the periphery of the counting wheel 8 is moved to a stop position between two teeth 12 of the gear ring 11 of the cap 7, which means that a subsequent clockwise rotation of the control wheel 9 does not act on the counting wheel 8, since clockwise rotation of the counting wheel 8 is prevented by the fact that the outer stop edge 26 of one of the locking tongues 25 of the counting wheel bears against one of the teeth 12 of the cap 7 and prevents clockwise rotation.

In the examples shown in the figures, the position of the rod-shaped pin 32 is such that the control wheel 9 rotates clockwise when the spray container 4 is pressed into the inhaler 1 upon administration of a spray dose. However, this is not a necessary condition for the invention. It is therefore possible to envisage the pin 32 being positioned instead on the diametrically opposite side of the control wheel 9. Such positioning would mean that the control wheel 9 first rotated anti-clockwise when the spray container 4 was pressed into the inhaler 1. Of course, such an arrangement would additionally mean that the direction of rotation, like the direction of the different gear rings 11, 22 and stop tongues 25, 30, would be opposite to those directions described above.

A counting mechanism 2 according to the invention can advantageously be produced by injection moulding of plastic. It is of course also possible to envisage combinations of plastic components and other materials, or counting mechanisms made completely or partially of metal.

Although the counting mechanism has been described in association with a spray dose inhaler, it is of course possible to use the counting mechanism in all contexts where a substance is to be sprayed in doses. For example, the counting mechanism can be used for spraying cosmetics or the like. In addition, spray dose inhalers in which the dosed substance is administered into the nose are of course also intended to come within the scope of the invention as defined by the appended claims.

## Claims

1. Counting mechanism (2) for counting the number of doses delivered from a container (4) and comprising means for connection to the container (4), **characterized in that** the counting mechanism (2) comprises a cap (7), a counting wheel (8), and a control wheel (9), all of which have a substantially circular shape and are concentric in relation to each other, the cap (7) having an outer surface and an inner surface, and a flange-like cap edge (13) projecting from the periphery of the inner surface, and comprising a gear ring (11) which is arranged inside the flange-like cap edge (13) on the inner surface of the cap (7), an indicator window (10) in the form of a through- opening in the cap (7), two concentrically arranged inner flanges (15, 17) which project centrally from the inner surface of the cap (7) and of which a first inner flange (15) is arranged radially inside a second inner flange (17) and projects a distance beyond the second inner flange (17), and **in that** the first inner flange (15) has a radially projecting locking tooth (16), and the counting wheel (8) has an outer surface and an inner surface and is arranged directly next to the cap (7) with the outer surface of the counting wheel (8) directed towards the inner surface of the cap (7), the counting wheel (8) comprising a centrally arranged through-hole (20) with a shape and size corresponding to the shape and size of the second inner flange (17) of the cap (7), at least one stop tongue (25) which projects from the periphery of the counting wheel and is arranged to engage in and cooperate with the gear ring (11) arranged on the inner surface of the cap (7), and a gear ring (22) arranged on the inner surface of the counting wheel (8), near the periphery of the counting wheel (8), and a measurement scale (21) arranged on the outer surface and placed so that it is visible through the indicator window (10), and **in that** the control wheel (9) has an outer surface and an inner surface and is arranged directly next to the counting wheel (8), with the outer surface of the control wheel (9) directed towards the inner surface of the counting wheel (8), the control wheel (9) comprising a centrally arranged through-hole (34) with a shape and size corresponding to the shape and size of the first inner flange (15) of the cap (7) and having a recess (35) arranged on the periphery of the hole (34), which recess (35) has a shape which substantially corresponds to the shape of the locking tooth (16) arranged on the cap (7) and with a size which slightly exceeds the size of the locking tooth (16), so that a predetermined clearance exists between the locking tooth (16) and the edges of the recess (35), at least one stop tongue (30) which projects from the periphery of the control wheel (9) and which is arranged to engage in and cooperate with the gear ring (22) arranged on the inner surface of the counting wheel (8), and a rod-shaped pin (32) which is arranged at right angles to the inner surface of the control wheel (9) and is connected to the control wheel (9) via a spring member (33), and **in that** the stop tongue (30) of the control wheel (9) is oriented in a tangential direction which is opposite to the direction of the gear ring (22) and stop tongue (25) of the counting wheel (8), and **in that** the direction of the teeth (12) in the gear ring (11) of the cap (7) coincides with the tangential direction of the stop tongue (30) of the control wheel (9), and furthermore **in that** the number of stop tongues (25, 30) and the number of teeth (12, 23) are identical for the counting wheel (8) and for the control wheel (9).

2. Counting mechanism according to Claim 1, **characterized in that** the number of stop tongues (25, 30) on the periphery of the counting wheel (8) and of the control wheel (9) is a prime number, and **in that** the stop tongues (25, 30) are symmetrically distributed about the periphery of the respective wheel (8, 9).

3. Counting mechanism according to Claim 1 or 2, **characterized in that** the first and second inner flange (15, 17) of the cap (7), and the centrally arranged through-holes (20, 34) in the counting wheel (8) and the control wheel (9), are substantially circular with a radius corresponding with the radius of the first and second inner flange (15, 17), respectively, of the cap (7).

4. Counting mechanism according to Claim 1, 2 or 3, **characterized in that** the stop tongues (25, 30) on the counting wheel (8) and the control wheel (9) are arranged to spring in the radial direction.

5. Counting mechanism according to any of Claims 1 to 4, **characterized in that** the number of teeth (12, 23) on the counting wheel (8) and the control wheel (9) is 46, and **in that** the number of stop tongues (25, 30) on the counting wheel (8) and the control wheel (9) is 3, and **in that** the clearance between the locking tooth (16) and the recess (35) is approximately 3°.

6. Counting mechanism according to Claim 1, **characterized in that** the spring member (33) is a flat spring.

7. Spray dose inhaler (1) with an inside and an outside, comprising a first tubular part (3) into which a spray container (4) can be introduced, a second tubular mouthpiece part (5) which is angled in relation to the first part (3) and which emerges into an opening (6) from which the contents of the spray container (4) can be delivered, and comprising a counting mechanism (2), according to claim 1 arranged substantially on the outside of the inhaler (1), where in the outside of the spray dose inhaler (1) is provided with coupling members (40, 43, 45, 47) for connecting the counting mechanism (2) to the spray dose inhaler (1), the coupling members comprising a through-hole (47) through which the rod-shaped pin (32) of the counting mechanism (2) can be introduced, the hole (47) being positioned so that the rod-shaped pin (32) is in contact with the spray container and is acted upon and deflected and/or changes position when the spray container (4) moves in the first part (3) of the spray dose inhaler (1).

## Patentansprüche

1. Zählvorrichtung (2) zum Zählen der Anzahl von von einem Behälter (4) abgegebenen Dosen, und mit Einrichtungen zur Verbindung an dem Behälter (4), **dadurch gekennzeichnet, dass** die Zählvorrichtung (2) Folgendes aufweist: eine Kappe (7), ein Zählrad (8) und ein Steuerrad (9), von denen alle eine im Wesentlichen kreisförmig Form aufweisen und in Bezug aufeinander konzentrisch ausgebildet sind, wobei die Kappe (7) eine äußere Fläche und eine innere Fläche, und einen flanschähnlichen Rand (13) aufweist, der von dem Umfang der inneren Fläche hervorsteht, und Folgendes aufweist: einen Zahnkranz (11), welcher innerhalb des flanschähnlichen Kappenrandes (13) auf der inneren Fläche der Kappe (7) angeordnet ist, ein Anzeigefenster (10) in der Form einer Durchgangsöffnung in der Kappe (7), zwei konzentrisch angeordnete innere Flansche (15, 17), die zentral von der inneren Fläche der Kappe (7) hervorstehen, und von welchen ein erster innerer Flansch (15) radial innerhalb eines zweiten inneren Flansches (17) angeordnet ist und eine Strecke über dem zweiten inneren Flansch (17) hervorsteht, und dass der erste innere Flansch (15) einen radial hervorstehenden Sperrzahn (16) aufweist, und das Zählrad (8) eine äußere Fläche und eine innere Fläche aufweist und mit der äußeren Fläche des Zählrads (8) zur inneren Fläche der Kappe (7) hin ausgerichtet direkt gleich neben der Kappe (7) angeordnet ist, wobei das Zählrad (8) Folgendes aufweist: ein zentral angeordnetes Durchgangsloch (20) mit einer Gestalt und Größe, die zu der Gestalt und Größe des zweiten inneren Flansches (17) der Kappe (7) korrespondiert, mindestens eine Rastzunge (25), welche vom Umfang des Zählrades hervorsteht und zum Eingriff und zum Zusammenwirken mit dem Zahnkranz (11) auf der inneren Fläche der Kappe (7) angeordnet ist, und einen Zahnkranz (22), der auf der inneren Fläche des Zählrads (8) angeordnet ist, und eine Maßskala (21), die auf der äußeren Fläche angeordnet und so platziert ist, dass sie durch das Anzeigefenster (10) zu sehen ist, und dass das Steuerrad (9) eine äußere Fläche und eine innere Fläche aufweist, welche auf die innere Fläche des Zählrads (8) hin ausgerichtet ist, wobei das Steuerrad (9) ein zentral angeordnetes Durchgangsloch (34) mit einer Gestalt und Größe aufweist, welche zu der Gestalt und Größe des ersten inneren Flansches (15) der Kappe (7) korrespondieren, und welches eine am Umfang des Loches (34) angeordnete Ausnehmung (35) aufweist, wobei die Ausnehmung (35) eine Form hat, die im Wesentlichen zu der Form des auf der Kappe (7) angeordneten Sperrzahns (16) korrespondiert, und mit einer Größe, die etwas über die Größe des Sperrzahns (16) hinausgeht, so dass ein vorher festgelegter Zwischenraum zwischen dem Sperrzahn (16) und den Kanten der Ausnehmung (35) besteht, und das Steuerrad (9) Folgendes aufweist: mindestens eine Rastzunge (30), die von dem Umfang des Steuerrads (9) hervorsteht, und welche so angeordnet ist, dass sie im Eingriff und im Zusammenwirken mit dem Zahnkranz (22) steht, der auf der inneren Fläche des Zählrads (8) angeordnet ist, und einen stabförmigen Stift (32), der im rechten Winkel zu der inneren Fläche des Steuerrads (9) angeordnet und mit dem Steuerrad (9) über ein Federelement (33) verbunden ist, und dass die Rastzunge (30) des Steuerrads (9) in einer tangentialen Richtung ausgerichtet ist, welche umgekehrt zu der Richtung des Zahnkranzes (22) und der Rastzunge (25) des Zählrads (8) liegt, und dass die Richtung der Zähne ( 12) in dem Zahnkranz (11) der Kappe (7) mit der tangentialen Richtung der Rastzunge (30) des Steuerrads (9) übereinstimmt, und dass weiterhin die Anzahl der Rastzungen (25, 30) und die Anzahl der Zähne (12, 23) bei dem Zählrad (8) und dem Steuerrad (9) identisch ist.

2. Zählvorrichtung (2) nach Anspruch 1, **dadurch gekennzeichnet, dass** die Anzahl der Rastzungen (25, 30) auf dem Umfang des Zählrads (8) und des Steuerrads (9) eine Primzahl ist, und dass die Rastzungen (25, 30) symmetrisch auf dem Umfang des jeweiligen Rads (8, 9) verteilt sind.

3. Zählvorrichtung (2) nach Anspruch 1 oder 2, **dadurch gekennzeichnet, dass** der erste und zweite innere Flansch (15, 17) der Kappe (7) und die in dem Zählrad (8) und dem Steuerrad (9) zentral angeordneten Durchgangslöcher im Wesentlichen kreisförmig mit einem Radius ausgebildet sind, der jeweils mit dem Radius des ersten und zweiten inneren Flansches (15, 17) der Kappe (7) korrespondiert.

4. Zählvorrichtung (2) nach Anspruch I, 2 oder 3, **dadurch gekennzeichnet, dass** die Rastzungen (25, 30) auf dem Zählrad (8) und dem Steuerrad (9) zum Federn in die radiale Richtung angeordnet sind.

5. Zählvorrichtung (2) nach einem der Ansprüche 1 bis 4, **dadurch gekennzeichnet, dass** die Anzahl der Zähne (12, 23) auf dem Zählrad (8) und dem Steuerrad (9) 46 beträgt, und dass die Anzahl der Rastzungen (25, 30) auf dem Zählrad (8) und dem Steuerrad (9) 3 beträgt, und dass der Zwischenraum zwischen dem Sperrzahn (16) und der Ausnehmung (35) ungefähr 3° beträgt.

6. Zählvorrichtung (2) nach Anspruch 1, **dadurch gekennzeichnet, dass** das Federelement (33) eine flache Feder ist.

7. Spraydosisinhalator (1) mit einer Innenseite und einer Außenseite, welcher ein erstes röhrenförmiges Teil (3), in welchen ein Spraybehälter (4) eingesetzt werden kann, und ein zweites röhrenförmiges Mundstückteil (5) aufweist, das in Bezug auf das erste Teil (3) abgewinkelt ausgebildet ist und in eine Öffnung (6) übergeht, aus welcher der Inhalt des Spraybehälters (4) ausgebracht werden kann, und wobei der Spraydosisinhalator eine Zählvorrichtung (2) nach Anspruch 1 aufweist, die im Wesentlichen an der Außenseite des Inhalators (1) angeordnet ist, wobei die Außenseite des Spraydosisinhalators (1) mit Kupplungselementen (40, 43, 45, 47) zur Verbindung der Zählvorrichtung (2) mit dem Spraydosisinhalator (1) versehen ist, wobei die Kupplungselemente ein Durchgangsloch (47) aufweisen, durch welches der stabförmige Stift (32) der Zählvorrichtung (2) hindurch geführt werden kann, wobei das Loch (47) so angeordnet ist, dass der stabförmige Stift (32) mit dem Spraybehälter in Kontakt steht und betätigt und durchgebogen wird und/oder seine Stellung verändert, wenn sich der Spraybehälter (4) in dem ersten Teil (3) des Spraydosisinhalators (1) bewegt.

## Revendications

1. Mécanisme (2) de comptage du nombre de doses distribuées par un récipient (4), comprenant un dispositif de raccordement du récipient (4), **caractérisé en ce que** le mécanisme (2) de comptage comporte un capuchon (7), une roue (8) de comptage et une roue (9) de commande, tous ces éléments ayant une forme pratiquement circulaire et étant concentriques les uns aux autres, le capuchon (7) ayant une surface externe et une surface interne, et un bord (13) en forme de flasque du capuchon dépassant de la périphérie de la surface interne, et comprenant une couronne dentée (11) disposée à l'intérieur du bord (13) en forme de flasque du capuchon à la surface interne du capuchon (7), une fenêtre (10) d'indicateur sous forme d'une ouverture qui débouche dans le capuchon (7), de flasques internes (15, 17) disposés concentriquement qui dépassent au centre de la surface interne du capuchon (7) et parmi lesquels un premier flasque interne (15) est disposé radialement à l'intérieur d'un second flasque interne (17) et dépasse d'une certaine distance au-delà du second flasque interne (17), **en ce que** le premier flasque interne (15) a une dent (16) de blocage qui dépasse radialement, et la roue de comptage (8) a une surface externe et une surface interne et est disposée directement près du capuchon (7) avec la surface externe de la roue de comptage (8) dirigée vers la surface interne du capuchon (7), la roue de comptage (8) comprenant un trou débouchant (20) disposé au centre et ayant une forme et une dimension qui correspondent à la forme et à la dimension du second flasque interne (17) du capuchon (7), au moins une languette (25) d'arrêt qui dépasse de la périphérie de la roue de comptage et qui est destinée à venir coopérer avec la couronne dentée (11) placée à la surface interne du capuchon (7) en pénétrant dans celle-ci, et une couronne dentée (22) disposée à la surface interne de la roue de comptage (8), près de la périphérie de la roue de comptage (8), et une graduation (21) de mesure placée à la surface externe est disposée afin qu'elle soit visible par la fenêtre (10) d'indicateur, **en ce que** la roue de commande (9) a une surface externe et une surface interne et est disposée directement à proximité de la roue de comptage (8), la surface externe de la roue de commande (9) étant dirigée vers la surface interne de la roue de comptage (8), la roue de commande (9) comprenant un trou débouchant (34) placé au centre et ayant une forme et une dimension qui correspondent à la forme et à la dimension du premier flasque interne (15) du capuchon (7) et ayant un évidement (35) placé à la périphérie du trou (34), cet évidement (35) ayant une forme qui correspond pratiquement à la forme de la dent (16) de blocage disposée sur le capuchon (7) et ayant une dimension légèrement supérieure à la dimension de la dent (16) de blocage, si bien qu'il existe un jeu prédéterminé entre la dent de blocage (16) et les bords de l'évidement (35), au moins une dent d'arrêt (30) qui dépasse de la périphérie de la roue de commande (9) et qui est disposée afin qu'elle coopère avec la couronne dentée (22) placée à la surface interne de la roue de comptage (8) et qui pénètre dans cette couronne, et une broche (32) en forme de tige disposée perpendiculairement à la surface interne de la roue de commande (9) et qui est raccordée à la roue de commande (9) par un organe à ressort (33), **en ce que** la languette d'arrêt (30) de la roue de commande (9) est orientée en direction tangente, en sens opposé à la direction de la couronne dentée (22) et de la languette d'arrêt (25) de la roue de comptage (8), et **en ce que** la direction des dents (12) de la couronne dentée (11) du capuchon (7) coïncide avec la direction tangente de la languette d'arrêt (30) de la roue de commande (9), et, en outre, le nombre de languettes d'arrêt (25, 30) et le nombre de dents (12, 23) sont identiques pour la roue de comptage (8) et la roue de commande (9).

2. Mécanisme de comptage selon la revendication 1, **caractérisé en ce que** le nombre de languettes d'arrêt (25, 30) placées à la périphérie de la roue de comptage (8) et de la roue de commande (9) est un nombre premier, et **en ce que** les languettes d'arrêt (25, 30) sont réparties symétriquement à la périphérie de la roue respective (8, 9).

3. Mécanisme de comptage selon la revendication 1 ou 2, **caractérisé en ce que** les premier et second flasques internes (4, 17) du capuchon (7) et les trous débouchants (20, 34) disposés au centre dans la roue de comptage (8) et la roue de commande (9) sont pratiquement circulaires avec un rayon qui correspond au rayon des premier et second flasques internes (15, 17) respectivement du capuchon (7).

4. Mécanisme de comptage selon la revendication 1, 2 ou 3, **caractérisé en ce que** les languettes d'arrêt (25, 30) de la roue de comptage (8) et de la roue de commande (9) sont disposées afin qu'elles présentent un effet de ressort en direction radiale.

5. Mécanisme de comptage selon l'une quelconque des revendications 1 à 4, **caractérisé en ce que** le nombre de dents (12, 23) sur la roue de comptage (8) et la roue de commande (9) est égal à 46, **en ce que** le nombre de languettes d'arrêt (25, 30) formées sur la roue de comptage (8) et la roue de commande (9) est égal à 3, et **en ce que** le jeu entre la dent de blocage (16) et l'évidement (35) est d'environ 3.

6. Mécanisme de comptage selon la revendication 1, **caractérisé en ce que** l'organe à ressort (33) est un ressort plat.

7. Inhalateur (1) de doses de pulvérisation ayant une partie interne et une partie externe, comprenant une première partie tubulaire (3) dans laquelle peut être introduit un récipient (4) de pulvérisation, une seconde partie tubulaire (5) d'embouchure qui forme un angle avec la première partie (3) et qui débouche dans une ouverture (6) qui permet la distribution du contenu du récipient (4) de pulvérisation, et comprenant un mécanisme de comptage (2) selon la revendication 1, dans lequel l'extérieur de l'inhalateur (1) de l'eau de pulvérisation comporte des organes d'accouplement (40, 43, 45, 47) destinés à raccorder le mécanisme de comptage (2) à l'inhalateur (1) de dose de pulvérisation, les organes d'accouplement comprenant un trou débouchant (47) par lequel la broche (32) en forme de tige du mécanisme de comptage (2) peut être introduite, le trou (47) étant disposé de manière que la broche (32) en forme de tige soit au contact du récipient de pulvérisation et subisse une action et fléchisse et/ou change de position lorsque le récipient (4) de pulvérisation se déplace dans la première partie (3) de l'inhalateur (1) de dose pulvérisée.
